**Europäisches Patentamt**

**European Patent Office**

**Office européen des brevets**

(19)

(11) Veröffentlichungsnummer: **0 221 971 B1**

(12) **EUROPÄISCHE PATENTSCHRIFT**

(45) Veröffentlichungstag der Patentschrift:
16.08.89

(51) Int. Cl.⁴: **B 60 H 3/06**

(21) Anmeldenummer: **86903294.6**

(22) Anmeldetag: **19.04.86**

(86) Internationale Anmeldenummer:
**PCT/EP 86/00239**

(87) Internationale Veröffentlichungsnummer:
**WO 86/06331 (06.11.86 Gazette 86/24)**

(54) **VORRICHTUNG ZUR ERFASSUNG VON SCHADSTOFFEN IN DER EINEM AUFENTHALT VON PERSONEN DIENENDEN KABINEN ZUGEFÜHRTEN LUFT.**

(30) Priorität: **23.04.85 DE 3514588**
**22.05.85 DE 3518320**
**26.06.85 DE 3522834**
**12.02.86 DE 3604340**

(43) Veröffentlichungstag der Anmeldung:
**20.05.87 Patentblatt 87/21**

(45) Bekanntmachung des Hinweises auf die Patenterteilung:
**16.08.89 Patentblatt 89/33**

(84) Benannte Vertragsstaaten:
**AT BE CH DE FR GB IT LI LU NL SE**

(56) Entgegenhaltungen:
**EP-A- 0 042 287**
**EP-A- 0 157 237**
**EP-A- 0 157 247**
**WO-A-85/01704**
**DE-A- 3 100 681**
**DE-A- 3 304 324**
**US-A- 2 899 282**

(73) Patentinhaber: **Hölter, Heinz, Dipl.-Ing.,**
**Beisenstrasse 39-41, D-4390 Gladbeck (DE)**

(72) Erfinder: **IGELBÜSCHER, Heinrich,**
**Marcq-en-Baroeul-Strasse 60, D-4390 Gladbeck (DE)**
Erfinder: **GRESCH, Heinrich, Franz-Lehàr-Strasse 25,**
**D-4600 Dortmund-Wickede (DE)**
Erfinder: **DEWERT, Heribert, Bahnhofstrasse 23,**
**D-4390 Gladbeck (DE)**

(74) Vertreter: **Spalthoff, Adolf, Dipl.-Ing.,**
**Pelmanstrasse 31 Postfach 34 02 20,**
**D-4300 Essen 1 (DE)**

ACTORUM AG

**Beschreibung**

Die Erfindung betrifft eine Vorrichtung zur Erfassung von Schadstoffen in der einem Aufenthalt von Personen dienenden Kabinen, vorzugsweise eines Kraftfahrzeuges, zugeführten Luft, unter Verwendung eines oder mehrer Sensoren und zur Anzeige und/oder Steuerung und/oder Regelung von Filtereinrichtungen und/oder Lüftungssystemen in Abhängigkeit von dem bzw. von den Sensoren ermittelten Meßwert bzw. Meßwerten.

Um die in der einem Aufenthalt von Personen dienenden Kabinen zugeführten Luft enthaltenen Schadstoffe zu beseitigen ist es erforderlich, diese zunächst zu erkennen, um dann Signale und/oder Stellgrößen zu erzeugen, um Filterreinigungen und/oder Lüftungssysteme ein- bzw. auszuschalten.

Es ist bereits vorgeschlagen worden, zur Erfassung von Schadstoffen in der Luft Analysengeräte vorzusehen, welche jedoch in ihrer Anschaffung und in ihrem Betrieb vergleichsweise aufwendig sind, ganz abgesehen davon, daß diese nur in der Lage sind, einzelne Schadstoffkomponenten zu erfassen.

Weiterhin ist es bekannt, Halbleitersensoren für diese Zwecke zu verwenden, welche im allgemeinen die Forderung erfüllen, auf die üblichen Luftverunreinigungen anzusprechen. Der Arbeitsbereich dieser Sensoren liegt in bezug auf ihre Ansprechempfindlichkeit jedoch wesentlich über der für den Menschen noch unbedenklichen Konzentration vieler Schadstoffe. Außerdem sind Halbleitersensoren empfindlich gegen Temperaturschwankungen und unterschiedliche Strömungsgeschwindlichkeiten der zu überwachenden Luftströme. Weiterhin sind die Ansprechzeiten dieser Sensoren besonders im Bereich niedrigerer Konzentrationen und ihre Dekontaminierungszeiten zu hoch, um beispielsweise zur Steuerung von Filtereinrichtungen und/oder Lüftungssystemen in Kraftfahrzeugkabinen Verwendung finden zu können.

Von diesem Stand der Technik ausgehend liegt der Erfindung die Aufgabe zugrunde, unter Vermeidung vorerwähnter Nachteile eine wirtschaftlich anspruchslose und wartungsfreie Vorrichtung mit geringem Raumbedarf zu schaffen, welche auf alle üblicherweise vorkommenden Luftverunreinigungen anspricht und eine zuverlässige Meldung gewährleistet.

Erfindungsgemäß wird dies dadurch erreicht, daß der Sensor sich in einem Gehäuse befindet und dessen Anströmseite innerhalb desselben eine Meßöffnung zum Durchtritt der zu überwachenden Luftströme vorgelagert ist, welche in eine Luftkammer des Gehäuses einmündet, in der ein Pulsator angeordnet ist, wobei in den dem Sensor abgewandten Zylinderraum der Luftkammer eine oder mehrere Lufteintrittsöffnungen einmünden und aus dem dem Sensor zugewandten Zylinderraum der Luftkammer eine oder mehrere Luftaustrittsöffnungen ausmünden. Durch diese Ausgestaltung wird erreicht, daß dem Sensor immer nur eine geregelte Luftmenge zugeführt wird, so daß dieser unabhängig von Änderungen der Strömungsgeschwindigkeiten die gewünschten Ergebnisse liefern kann. Das verwendete Schwingpumpensystem sichert eine konstante Luftbespülung des Sensors und führt zur Verkürzung der Dekontaminierungszeiten. Außerdem beaufschlagt ein weitgehend vergleichmäßigter, konstanter Luftstrom den Sensor.

Im Bereich der Abströmseite des Sensors befinden sich in einer Gehäusewandung Luftaustrittskanäle, aus denen der Teil der in das Gehäuse eintretenden Luftmenge abströmen kann, welcher der Beaufschlagung des Sensors gedient hat.

Der Pulsator ist von einem Kolben gebildet, welcher mit einem Schwinger gekoppelt ist, wobei der Schwinger vorteilhaft den dem Sensor abgewandten Gehäuseabschluß bildet, so daß sich insgesamt eine sehr kompakte Baueinheit ergibt.

Gemäß einem weiteren Merkmal der Erfindung weist der Kolben in unterschiedlicher Strömungsrichtung unterschiedliche Strömungswiderstände auf. Wird der Kolben in Schwingung versetzt, so erzeugt dieser eine gerichtete Luftströmung, welche auf den Sensor hin gerichtet ist. Zu diesem Zwecke ist der Kolben an seiner Außenseite mit einem sägezahnartigen Profil ausgerüstet, derart, daß die erweiterten Bereiche in Richtung auf den Sensor hin ausgerichtet sind. Durch die Schwingung des Kolbens wird somit eine vorbestimmte Luftströmung erzeugt, wobei gleichzeitig das Ansaugen der zu überwachenden Luft in das Gehäuse bewirkt wird.

Die Lufteintritts- und Luftaustrittsöffnungen sind in ihren Durchtrittsquerschnitten asymmetrisch ausgebildet. Diese erweitern sich beispielsweise von der Luftkammer nach außen hin diffusorartig. Dadurch wird erreicht, daß bei der durch den Schwinger verursachten Luftpulsierung genügend Luft in das Gehäuse eintritt, um eine ausreichend hohe Bespülung des Sensors sicherzustellen.

Vorteilhaft ist in der Luftkammer eine Luftkonditionierung, beispielsweise in Form eines Lufterwärmers, vorgesehen. Diese befindet sich in der Luftkammer im Bereich zwischen dem Pulsator und der Meßöffnung. Durch diese Konditionierung wird eine gleichmäßige Temperierung der dem Sensor zugeführten Luftmengen erreicht, so daß dieser in seinem optimalen Ansprechbereich beaufschlagt wird. Der Sensor liefert daher ein weitestgehend temperaturunabhängiges Signal. Auf diese Weise wird die Sensorkennlinie im unteren Arbeitsbereich so linearisiert, daß auch im unteren Konzentrationsbereich Schadstoffmeldungen durchgeführt werden können.

Gemäß einem weiteren Merkmale der Erfindung ist dem Pulsator eine Heizeinrichtung zugeordnet. Diese befindet sich vorteilhaft in der Gehäusewandung und/oder im Bereich zwischen Gehäusewandung und dem Pulsator und/oder an der Außenseite des Pulsators. Durch diese zusätzliche Heizeinrichtung, beispielsweise in Form eines elektrischen Heizdrahtes, wird eine zusätzliche Erwärmung der zu überwachenden Luftmengen bereits im Bereich des Schwingkolbens erreicht, was sich insbesondere für die Wintermonate als sehr vorteilhaft erweist.

Gemäß einem weiteren Merkmal der Erfindung ist der Sensor in einem druckfesten Gehäuse angeordnet, in welchem eine vorkonditionierte Luftmenge komprimiert dem Sensor zugeführt wird, so daß auch geringste Spuren von Geruchsstoffen vom Sensor meßtechnisch erfaßt werden und zur Erzeugung einer Anzeige und/oder eines Stellimpulses dienen.

Nach einem weiteren Merkmal der Erfindung wird die Luftsäule vor der Anströmseite des Sensors in Schwingungen versetzt, und zwar in eine hochfrequente Schwingung. Die auf die Anströmseite des Sensors einwirkende schwingende Luftsäule führt zu einem vergleichsweise sehr schnellen «Abspülen» der auf der Anströmseite des Sensors befindlichen Fremdstoffdotierung und damit zu einer erheblichen Steigerung der Dekontaminierungszeiten, wobei durch das Außengehäuse etwaige Beeinflussung durch äußere Anströmung ausgeschaltet ist.

Im Rahmen der Erfindung ist es auch möglich, die Anströmseite des Sensors aufzuheizen, um möglichst konstante Temperaturverhältnisse zu erzeugen.

Ein Ausführungsbeispiel der Erfindung ist an Hand der Zeichnung näher erläutert, und zwar zeigt:

Fig. 1 eine geschnittene Ansicht der Vorrichtung und

Fig. 2 in vergrößertem Maßstab einen Ausschnitt aus Figur 1, den Pulsator betreffend.

Mit 1 ist der Sensor bezeichnet, welcher sich in einem Gehäuse 2 befindet. Der Anströmseite des Sensors 1 vorgelagert ist innerhalb des Gehäuses 2 die Meßöffnung 9. Die Meßöffnung 9 mündet in die Luftkammer 5 des Gehäuses 2 ein. In dieser Luftkammer 5 ist ein Pulsator 7 angeordnet. In den dem Sensor 1 abgewandten Zylinderraum der Luftkammer 5 münden die Lufteintrittsöffnungen 3 ein. Aus dem dem Sensor 1 zugewandten Zylinderraum der Luftkammer 5 münden die Luftaustrittsöffnungen 4 aus. Im Bereich der Abströmseite des Sensors 1 befindet sich in der stirnseitigen Gehäusewandung ein Luftaustrittskanal 6.

Der Pulsator 7 ist von einem Kolben gebildet, welcher mit einem Schwinger 8 gekoppelt ist. Der Schwinger 8 bildet den dem Sensor 1 abgewandten Gehäuseabschluß. Als Schwinger 1 findet vorzugsweise ein Magnetfreischwinger Verwendung.

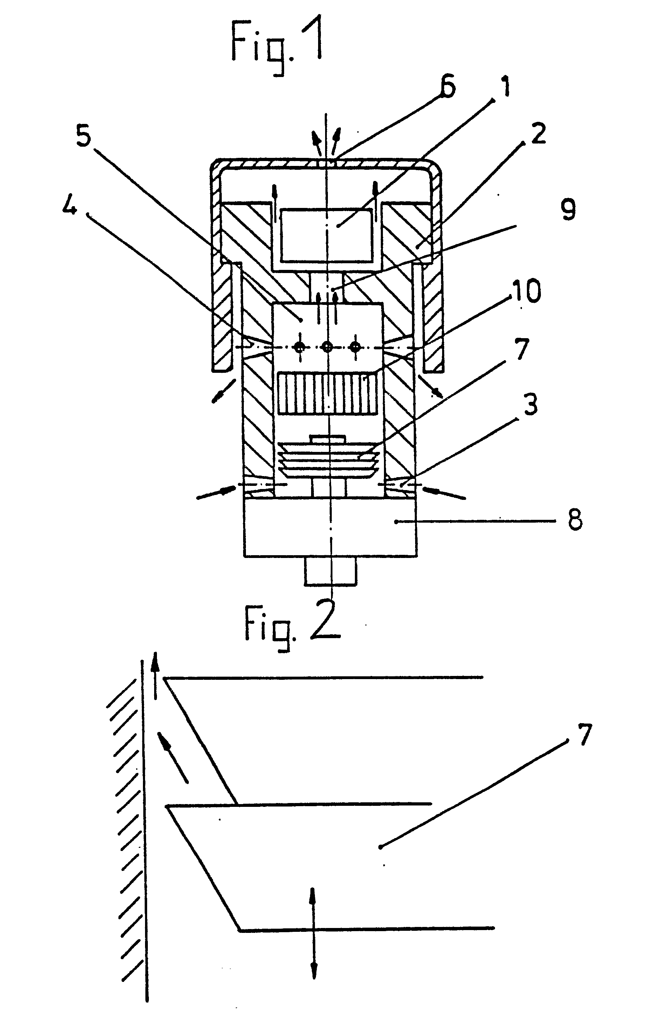

Wie insbesondere aus Figur 2 ersichtlich, ist der als Pulsator 7 dienende Kolben an seiner Außenseite mit einem sägezahnartigen Profil ausgerüstet, wobei die erweiterten Bereiche in Richtung auf den Sensor 1 hin ausgerüstet sind. Mit dem Doppelpfeil in Figur 2 ist die Schwingrichtung des Pulsators 7 angedeutet und durch die Pfeile der dem Sensor 1 zugeförderte Luftstrom.

Die Luftein- 3 und Luftaustrittsöffnungen 4 erweitern sich von der Luftkammer 5 nach außen hin diffusorartig.

In der Luftkammer 5 ist eine Luftkonditionierung 10 vorgesehen, welche sich im Bereich zwischen dem Pulsator 7 und der Meßöffnung 9 befindet.

Wie aus Gründen einer besseren Übersichtlichkeit halber nicht weiter dargestellt, kann dem Pulsator 7 eine Heizeinrichtung zugeordnet sein, beispielsweise in Form einer elektrischen Heizwendel, die in der benachbarten Wandung des Gehäuses 2, zwischen der Gehäusewandung und dem Pulsator 7 oder aber auch an der Außenseite des Pulsators im Bereich des sägezahnartigen Profils vorgesehen sein kann.

**Patentansprüche**

1. Vorrichtung zur Erfassung von Schadstoffen in der einem Aufenthalt von Personen dienenden Kabinen oder dgl., vorzugsweise eines Kraftfahrzeuges, zugeführten Luft, unter Verwendung eines oder mehrerer Sensoren (1) und zur Anzeige und/oder Steuerung und/oder Regelung von Filtereinrichtungen und/oder Lüftungssystemen in Abhängigkeit von dem bzw. den vom Sensor (1) bzw. den Sensoren ermittelten Meßwert bzw. Meßwerten, dadurch gekennzeichnet, daß der Sensor (1) sich in einem Gehäuse (2) befindet, dessen Anströmseite innerhalb desselben eine Meßöffnung (9) vorgelagert ist, welche in eine Luftkammer (5) des Gehäuses (2) einmündet, in der ein Pulsator (7) angeordnet ist, wobei in den dem Sensor (1) abgewandten Zylinderraum der Luftkammer (5) eine oder mehrere Lufteintrittsöffnungen (3) einmünden und aus dem dem Sensor (1) zugewandten Zylinderraum der Luftkammer (5) eine oder mehrere Luftaustrittsöffnungen (4) ausmünden.

2. Vorrichtung nach Anspruch 1, dadurch gekennzeichnet, daß sich im Bereich der Abströmseite des Sensors (1) in einer Gehäusewandung ein oder mehrere Luftaustrittskanäle (6) befinden.

3. Vorrichtung nach einem der vorhergehenden Ansprüche, dadurch gekennzeichnet, daß der Pulsator (7) von einem Kolben gebildet ist, welcher mit einem Schwinger (8) gekoppelt ist.

4. Vorrichtung nach einem der vorhergehenden Ansprüche, dadurch gekennzeichnet, daß der Schwinger (8) den dem Sensor (1) abgewandten Gehäuseabschluß bildet.

5. Vorrichtung nach einem der vorhergehenden Ansprüche, dadurch gekennzeichnet, daß der Kolben in unterschiedlicher Strömungsrichtung unterschiedliche Strömungswiderstände aufweist.

6. Vorrichtung nach einem der vorhergehenden Ansprüche, dadurch gekennzeichnet, daß der Pulsator (7) an seiner Außenseite mit einem sägezahnartigen Profil ausgerüstet ist, wobei die erweiterten Bereiche in Richtung auf den Sensor (1) hin ausgerichtet sind.

7. Vorrichtung nach einem der vorhergehenden Ansprüche, dadurch gekennzeichnet, daß die Lufteintritts- (3) und Luftaustrittsöffnungen (4) in ihren Durchtrittsquerschnitten asymmetrisch ausgebildet sind.

8. Vorrichtung nach einem der vorhergehenden Ansprüche, dadurch gekennzeichnet, daß die Lufteintritts- (3) und Luftaustrittsöffnungen (4) sich von der Luftkammer (5) nach außen hin diffusorartig erweitern.

9. Vorrichtung nach einem der vorhergehenden Ansprüche, dadurch gekennzeichnet, daß in der Luftkammer (5) eine Luftkonditionierung (10) vorzugsweise als Heizeinrichtung vorgesehen ist.

10. Vorrichtung nach einem vorhergehenden Ansprüche, dadurch gekennzeichnet, daß die Luftkonditionierung (10) sich in der Luftkammer (5) im Bereich zwischen dem Pulsator (7) und der Meßöffnung (9) befindet.

11. Vorrichtung nach einem der vorhergehenden Ansprüche, dadurch gekennzeichnet, daß dem Pulsator (7) eine Heizeinrichtung zugeordnet ist.

12. Vorrichtung nach einem der vorhergehenden Ansprüche, dadurch gekennzeichnet, daß die Heizeinrichtung des Pulsators (7) in der Gehäusewandung und/oder im Bereich zwischen Gehäusewandung und dem Pulsator (7) und/oder an der Außenseite des Pulsators (7) vorgesehen ist.

13. Vorrichtung nach einem der vorhergehenden Ansprüche, dadurch gekennzeichnet, daß der Sensor (1) in einem druckfesten Gehäuse angeordnet ist, in welchem eine vorkonditionierte Luftmenge komprimiert dem Sensor (1) zugeführt wird.

14. Vorrichtung nach einem der vorhergehenden Ansprüche, dadurch gekennzeichnet, daß die Luftsäule vor der Anströmseite des Sensors (1) in Schwingungen, vorzugsweise hochfrequente Schwingungen, versetzt wird.

15. Vorrichtung nach einem der vorhergehenden Ansprüche, dadurch gekennzeichnet, daß die Anströmseite des Sensors (1) beheizt ist.

## Claims

1. A device for detecting harmful substances in the air supplied to cabins or the like, preferably of a motor vehicle, which accomodate persons, the device using one or a plurality of sensors (1) and adapted to display and/or control and/or regulate filtering means and/or ventilation systems as a function of the measured value or values ascertained by the sensor (1) or sensors, characterised in that the sensor (1) is disposed in a housing (2), of which the active side within the housing has in front of it a measuring aperture (9) which discharges into an air chamber (5) of the housing (2) in which there is a pulsator (7), one or a plurality of air inlet apertures (3) opening out into that cylinder space of the air chamber (5) which is remote from the sensor (1) and one or a plurality of air outlet orifices (4) discharging from the cylinder space of the air chamber (5) which is facing the sensor (1).

2. A device according to Claim 1, characterised in that one or a plurality of air outlet passages (6) are disposed in the housing wall, in the region of the inactive side of the sensor (1).

3. A device according to one of the preceding Claims, characterised in that the pulsator (7) is constituted by a piston which is coupled to an oscillator (8).

4. A device according to one of the preceding Claims, characterised in that the oscillator (8) forms the end of the housing which is remote from the sensor (1).

5. A device according to one of the preceding Claims, characterised in that the piston has different flow resistances in different directions of flow.

6. A device according to one of the preceding Claims, characterised in that the pulsator (7) is on its outside equipped with a saw tooth-like profile, the widened out portions pointing in the direction of the sensor (1).

7. A device according to one of the preceding Claims, characterised in that the air inlet (3) and air outlet apertures (4) are asymmetrical in their through-flow cross-sections.

8. A device according to one of the preceding Claims, characterised in that the air inlet (3) and air outlet apertures (4) widen out diffusor-like outwardly from the air chamber (5).

9. A device according to one of the preceding Claims, characterised in that air conditioning (10), preferably as a heating means, is provided in the air chamber (5).

10. A device according to one of the preceding Claims, characterised in that the air conditioning (10) is disposed in the air chamber (5) in the region between the pulsator (7) and the measuring aperture (9).

11. A device according to one of the preceding Claims, characterised in that a heating means is associated with the pulsator (7).

12. A device according to one of the preceding Claims, characterised in that the heating means of the pulsator (7) is provided in the housing wall and/or in the region between the housing wall and the pulsator (7) and/or on the outside of the pulsator (7).

13. A device according to one of the preceding Claims, characterised in that the sensor (1) is disposed in a pressure-resistant housing in which a preconditioned quantity of air is compressed and fed to the sensor (1).

14. A device according to one of the preceding Claims, characterised in that the air column upstream of the active side of the sensor (1) is caused to oscillate, preferably with high-frequency oscillations.

15. A device according to one of the preceding Claims, characterised in that the active side of the sensor (1) is heated.

## Revendications

1. Dispositif pour mesurer des substances nocives dans l'air fourni à une cabine ou similaire servant au séjour de personnes, de préférence à une cabine de véhicule automobile, utilisant un ou plusieurs capteurs (1) et destiné à l'affichage et/ou à la commande et/ou à la régulation de dispositifs de filtration et/ou de systèmes d'aération en fonction de la valeur de mesure ou des valeurs de mesures déterminées par le ou les capteur(s), caractérisé en ce que le capteur (1) se trouve dans un carter (2) et qu'en avant de la face amont du capteur se trouve à l'intérieur dudit carter un orifice de mesure (9) débouchant dans une chambre à air (5) du carter (2) dans laquelle est disposé un pulsateur (7), un ou plusieurs orifices d'entrée d'air (3) débouchant dans l'espace cylindrique de la chambre à air (5) qui est opposé au capteur (1) et un ou plusieurs orifices de sortie d'air (4) sortant de l'espace cylindrique de la chambre à air (5) qui est tourné vers le capteur (1).

2. Dispositif selon la revendication 1, caractérisé en ce qu'un ou plusieurs canaux de sortie d'air (6) se trouvent dans la zone de la face aval du capteur (1), dans une paroi du carter.

3. Dispositif selon l'une des revendications précédentes, caractérisé en ce que le pulsateur (7) est formé par un piston qui est accouplé à un oscillateur (8).

4. Dispositif selon l'une des revendications précédentes, caractérisé en ce que l'oscillateur (8) forme la limite du carter opposée au capteur (1).

5. Dispositif selon l'une des revendications précédentes, caractérisé en ce que le piston présente des résistances à l'écoulement différentes dans différentes directions d'écoulement.

6. Dispositif selon l'une des revendications précédentes, caractérisé en ce que le pulsateur (7) est muni sur sa face extérieure d'un profil en dents de scie, les zones élargies étant orientées en direction du capteur (1).

7. Dispositif selon l'une des revendications précédentes, caractérisé en ce que les orifices d'entrée d'air (3) et de sortie d'air (4) sont réalisés de manière asymétrique dans leurs sections transversales de passage.

8. Dispositif selon l'une des revendications précédentes, caractérisé en ce que les orifices d'entrée d'air (3) et de sortie d'air (4) s'évasent en forme de diffuseur, allant de la chambre à air (5) vers l'extérieur.

9. Dispositif selon l'une des revendications précédentes, caractérisé en ce qu'il est prévu dans la chambre à air (5) un conditionnement d'air (10), de préférence sous forme de dispositif de chauffage.

10. Dispositif selon l'une des revendications précédentes, caractérisé en ce que le conditionnement d'air (10) se trouve dans la chambre à air (5), dans la zone située entre le pulsateur (7) et l'orifice de mesure (9).

11. Dispositif selon l'une des revendications précédentes, caractérisé en ce qu'un dispositif de chauffage est affecté au pulsateur (7).

12. Dispositif selon l'une des revendications précédentes, caractérisé en ce que le dispositif de chauffage du pulsateur (7) est prévu dans la paroi du carter et/ou dans la zone située entre la paroi du carter et le pulsateur (7) et/ou sur la face extérieure du pulsateur (7).

13. Dispositif selon l'une des revendications précédentes, caractérisé en ce que le capteur (1) est disposé dans un carter résistant à la pression, dans lequel un débit d'air préconditionné est amené au capteur (1) sous forme comprimée.

14. Dispositif selon l'une des revendications précédentes, caractérisé en ce que la colonne d'air située devant la face amont du capteur (1) est déplacée par vibrations, de préférence par vibrations à haute fréquence.

15. Dispositif selon l'une des revendications précédentes, caractérisé en ce que la face amont du capteur (1) est chauffée.

# Fig. 1

6
1
5
2
4
9
10
7
3
8

# Fig. 2

7